(19) Europäisches Patentamt
European Patent Office
Office européen des brevets

(11) Publication number: **0 115 400**
**B1**

(12) **EUROPEAN PATENT SPECIFICATION**

(45) Date of publication of patent specification: **26.03.86**

(21) Application number: **84300285.8**

(22) Date of filing: **18.01.84**

(51) Int. Cl.[4]: **C 07 D 403/06,**
C 07 D 249/08, A 61 K 31/41
// (C07D403/06, 249:08,
257:04),(C07D403/06, 249:08,
233:64)

# (54) Triazole antifungal agents.

(30) Priority: **21.01.83 GB 8301699**

(43) Date of publication of application:
**08.08.84 Bulletin 84/32**

(45) Publication of the grant of the patent:
**26.03.86 Bulletin 86/13**

(84) Designated Contracting States:
**AT BE CH DE FR GB IT LI LU NL SE**

(58) References cited:
**GB-A-2 078 719**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT LI LU NL SE AT**

(72) Inventor: **Richardson, Kenneth, Dr.**
**48 St. Stephens Hill**
**Canterbury Kent (GB)**
Inventor: **Gymer, Geoffrey Edward, Dr.**
**19 Palmer Road**
**Wingham Canterbury Kent (GB)**

(74) Representative: **Moore, James William**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 115 400 B1**

Courier Press, Leamington Spa, England.

**Description**

This invention relates to novel triazole derivatives which have antifungal activity and are useful in the treatment of fungal infections in animals, including humans.

British patent application number 2078719A and European patent application number 44605 disclose certain 1,3-bis-heterocyclyl-2-aryl-propan-2-ol derivatives wherein the heterocyclic group is a (1,2,4-triazol-1-yl) or (imidazol-1-yl) group. The compounds are stated to be useful as plant fungicides and growth regulators and also for the treatment of fungal diseases in humans and other animals.

According to the present invention there are provided 1-heterocyclyl-2-aryl-3-(1,2,4-triazol-1-yl)-propan-2-ol compounds wherein the heterocyclyl group is selected from particular imidazolyl, triazolyl or tetrazolyl groups which are linked by a ring carbon atom. We have found that these compounds are particularly effective as systemic agents for the treatment of fungal diseases in humans and other animals.

Thus, according to the invention, there are provided compounds of the formula:—

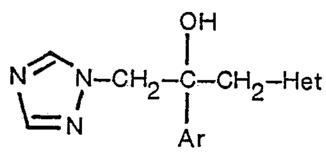

(I)

where

Ar is phenyl optionally substituted by 1 to 33 substituents each independently selected from F, Cl, Br, I, $CF_3$, $C_1$—$C_4$ alkyl and $C_1$—$C_4$ alkoxy; and

Het is a group of the formula:

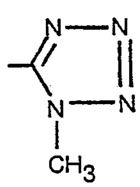
,
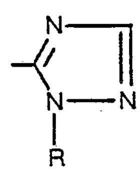
,
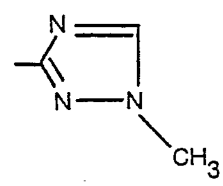
.

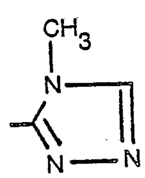
or
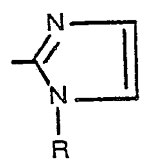

wherein R is H or $CH_3$;
and their pharmaceutically acceptable salts.

The invention also provides a pharmaceutical composition comprising a compound of the formula (I) or a pharmaceutically acceptable salt thereof, together with a pharmaceutically acceptable diluent or carrier.

The invention further provides a compound of the formula (I) or a pharmaceutically acceptable salt thereof, for use in treating fungal infections in animals, including humans.

Alkyl and alkoxy groups containing 3 or 4 carbon atoms may be straight or branched-chain.

When Ar is an optionally substituted phenyl group, it is preferably phenyl substituted by 1 to 3 substituents, more preferably 1 or 2 substituents, each independently selected from F, Cl, Br, I and $CF_3$. The most preferred individual groups represented by Ar are 4-fluorophenyl, 4-chlorophenyl, 4-bromophenyl, 4-iodophenyl, 4-trifluoromethylphenyl, 2-chlorophenyl, 2,4-dichlorophenyl, 2,4-difluorophenyl, 2,5-difluorophenyl, 2-chloro-4-fluoro 2,4,6-trifluorophenyl and 4-bromo-2,5-difluorophenyl; 2,4-dichlorophenyl is particularly preferred.

The preferred heterocyclic group Het is 1-methyltetrazol-5-yl.

A particularly preferred individual compound of the invention is 2-(2,4-dichloropheny)-1-(1-methyltetrazol-5-yl)-3-(1H-1,2,4-triazol-1-yl)propan-2-ol;

The compounds of formula (I) wherein Het is 1-methyl-tetrazolyl can be prepared according to the following reaction scheme wherein Ar is as previously defined.

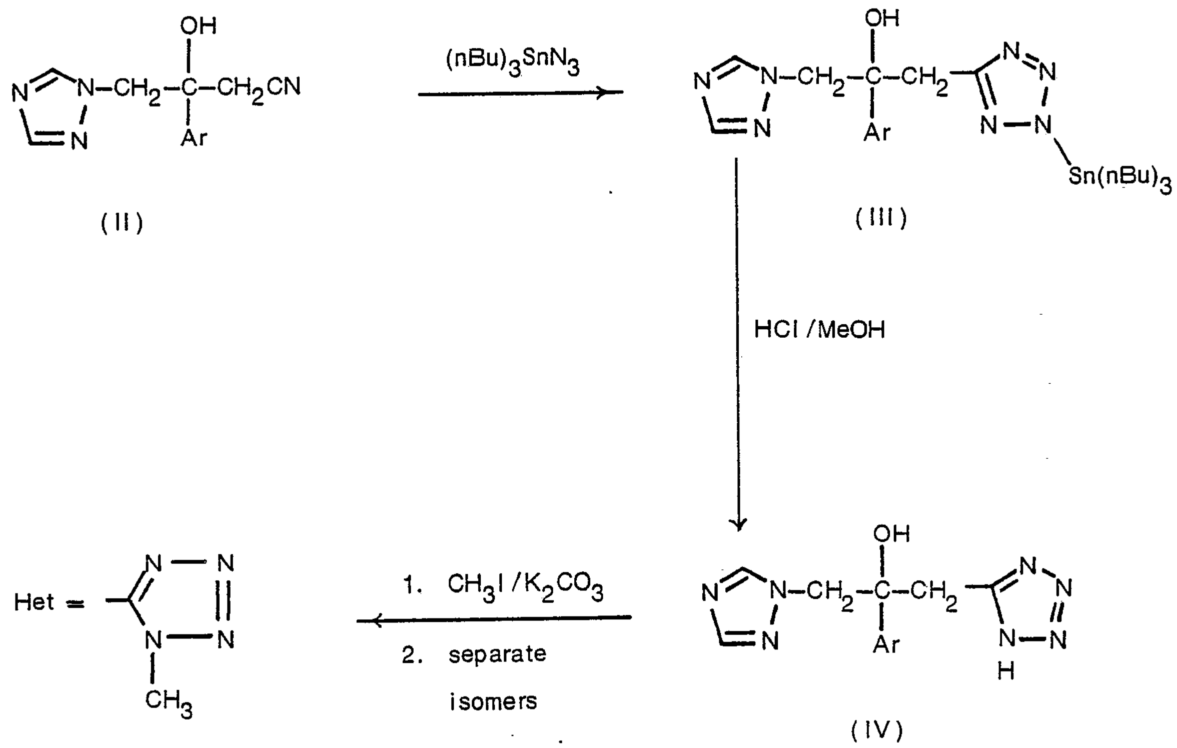


The reaction between the nitrile (II) and tri-n-butyltin azide is simply performed by heating the reactants together. A 10—20% molar excess of the azide is preferably used to ensure complete reaction. A temperature in the range of 100 to 180°C can be used. The period required for the reaction to go to completion will depend upon the precise nature of the reactants and the temperature employed but a period of three hours at 160°C will generally be sufficient. The reaction mixture is cooled and trituration with an organic solvent to remove unreacted azide provides the product (III). This is decomposed by suspending in a solution of methanolic hydrogen chloride at room temperature for several hours to provide the tetrazole derivative (IV).

The final methylation step is performed in a conventional manner by treating the tetrazole (IV) with methyl iodide and potassium carbonate in an inert organic solvent, e.g. N,N-dimethylformamide. A period of three hours at room temperature is generally sufficient to ensure complete reaction and the resulting mixture of the 1-methyl and 2-methyl-tetrazole isomers is separated by chromatography to yield the desired product.

The compounds of the formula (I) wherein Het is triazolyl or 1-, 2- or 4-methyltriazolyl may be prepared according to the following reaction scheme wherein Ar is as previously defined:

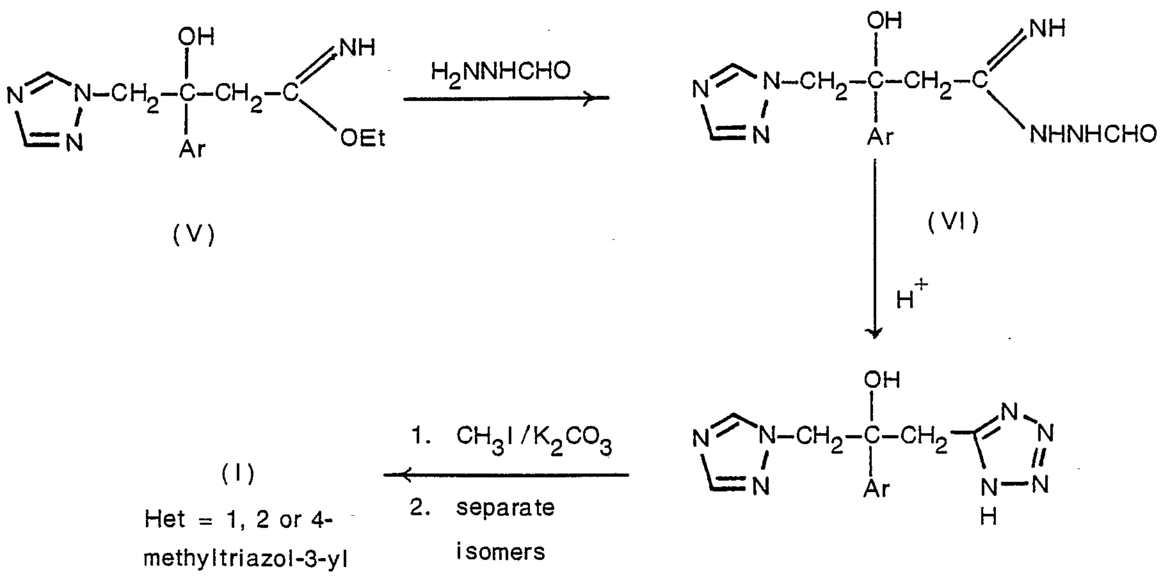

The reaction between the imino ether (V) and formyl hydrazine is performed by adding the reactants in equimolar proportions to an inert organic solvent, for example methylene chloride, a period of 12 hours at room temperature generally being sufficient to enable the reaction to go to completion. The intermediate (VI) need not be isolated but is heated in an inert organic solvent, e.g. toluene in the presence of a trace of acid, e.g. para-toluenesulphonic acid, to effect ring closure to provide the derivative of formula (I) wherein Het is 1,2,4-triazol-3-yl. The product is isolated in a conventional manner by washing and evaporation of the solvent and the crude product may be further purified, if required, by chromatography or by salt formation.

Compounds wherein Het is 1-, 2- or 4-methyl-triazolyl are readily prepared by methylation of the unsubstituted triazolyl derivative using a conventional methylation reaction, for example using methyl iodide and potassium carbonate as already described for the tetrazole derivative. In this case the product is obtained as a mixture of the three possible N-methyl isomers and these are separated by chromatography to yield the desired 1-, 2- and 4-methyl isomers.

The compounds of the formula (I) wherein Het is 2-imidazolyl may be prepared from the imino ether (V) by first reacting with aminoacetaldehyde as its diethyl acetal followed by treatment with acid to effect ring closure. The 1-methylimidazolyl derivative is again readily prepared by methylation of the unsubstituted compound, for example by reacting with methyliodide and potassium carbonate in acetone.

Pharmaceutically acceptable acid addition salts of the compounds of the formula (I) are those formed from strong acids which form non-toxic acid addition salts, such as hydrochloric, hydrobromic, sulphuric, oxalic and methanesulphonic acids.

The salts may be obtained by conventional procedures, e.g. by mixing solutions containing approximately equimolar amounts of the free base and desired acid, and the required salt is collected by filtration, if insoluble, or by evaporation of the solvent.

The compounds of the formula (I) and their pharmaceutically acceptable salts are antifungal agents, useful in combating fungal infections in animals, including humans. For example they are useful in treating topical fungal infections in man caused by, among other organisms, species of *Candida, Trichophyton, Microsporum* or *Epidermophyton,* or in mucosal infections caused by *Candida albicans* (e.g. thrush and vaginal candidiasis). They can also be used in the treatment of systemic fungal infections caused by, for example, *Candida albicans, Cryptococcus neoformans, Aspergillus fumigatus, Coccidioides, Paracoccidioides, Histoplasma* or *Blastomyces.*

The *in vitro* evaluation of the antifungal activity of the compounds can be performed by determining the minimum inhibitory concentration (m.i.c.) of the test compounds in a suitable medium at which growth of the particular micro-organism fails to occur. In practice, a series of agar plates, each having the test compound incorporated at a particular concentration is inoculated with a standard culture of, for example, *Candida albicans* and each plate is then incubated for 48 hours at 37°C. The plates are then examined for the presence or absence of growth of the fungus and the appropriate m.i.c. value is noted. Other microorganisms used in such tests can include *Cryptococcus neoformans, Aspergillus fumigatus, Trichophyton* spp; *Microsporum* spp; *Epidermophyton floccosum, Coccidioides immitis* and *Torulopsis glabrata.*

The *in vivo* evaluation of the compounds can be carried out at a series of dose levels by intraperitoneal or intravenous injection or by oral administration, to mice which are inoculated with a strain of *Candida albicans.* Activity is based on the survival of a treated group of mice after the death of an untreated group of mice following 48 hours observation. The dose level at which the compound provides 50% protection against the lethal effect of the infection is noted.

For human use, the antifungal compounds of the formula (I) can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules or ovules either alone or in admixture with excipients, or in the form of elixirs or suspensions containing flavouring or colouring agents. They can be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, the daily dosage level of the antifungal compounds of the formula (I) will be from 0.1 to 10 mg/kg (in divided doses) when administered by either the oral or parenteral route. Thus tablets or capsules of the compounds will contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case; there can, of course, be individual instances where higher or lower dosage ranges are merited, and such are within the scope of this invention.

Alternatively, the antifungal compounds of formula (I) can be administered in the form of a suppository or pessary, or they may be applied topically in the form of a lotion, solution, cream, ointment or dusting powder. For example, they can be incorporated into a cream consisting of an aqueous emulsion of polyethylene glycols or liquid paraffin; or they can be incorporated, at a concentration between 1 and 10%, into an ointment consisting of a white wax or white soft paraffin base together with such stabilizers and preservatives as may be required.

The compounds of the formula (I) and their salts also have activity against a variety of plant pathogenic fungi, including for example various rusts, mildews and moulds, and the compounds are thus useful as agricultural fungicides for treating plants and seeds to eradicate or prevent such diseases.

The following Examples illustrate the invention.

### Example 1

2-(2,4-Dichlorophenyl)-1-(1-methyltetrazol-5-yl)-3-(1H-1,2,4-triazol-1-yl)propan-2-ol

1. A paste of tri-n-butyltin azide (2.45 g) and 1-cyano-2-(2,4-dichlorophenyl)-3-(1H-1,2,4-triazol-1-yl)-propan-2-ol (2.0 g) was heated at 160°C for three hours. A further quantity of tri-n-butyltin azide (0.2 g) was added and the heating continued for a further hour to complete the reaction. The resulting viscous oil was triturated with ethyl acetate and the product collected and dried under vacuum to give 2-(2,4-dichlorophenyl)-1-(1H-1,2,4-triazol-1-yl)-3-(2-tribut-1-ylstannyltetrazol-5-yl)propan-2-ol as a crystalline solid (3.1 g, 73%) m/e 628.

2. The product from the first stage was suspended in methanol (100 ml) and a rapid stream of hydrogen chloride gas was passed through the solution for ten minutes. The solution was then stirred for three hours and the solvent was evaporated under reduced pressure. The residue was triturated with several portions of diisopropyl ether and the resulting white solid was crystallized from water to give 2-(2,4-dichlorophenyl)-1-(tetrazol-5-yl)-3-(1H-1,2,4-triazol-1-yl)propan-2-ol (1.6 g, 95%), m.p. 214—215°C. Found: C, 42.4; H, 3.4; N, 27.7. $C_{12}H_{11}Cl_2N_7O$ requires C, 42.3; H, 3.3; N, 28.8%.

3. A solution of the tetrazole product from the second stage (1.3 g) in N,N-dimethylformamide (25 ml) was stirred with methyl iodide (2.5 g) and excess potassium carbonate (1.3 g) at room temperature for three hours. The reaction mixture was then diluted with water (75 ml) and extracted with ethyl acetate (3 × 50 ml). The combined extracts were washed with brine (2 × 50 ml), dried over $MgSO_4$ and evaporated to give a mixture of the 1-methyl and 2-methyl isomers as a gum. Chromatography on silica, eluting with methylene chloride containing 1% (by volume) isopropanol gave two components. The fractions containing the second, more polar component were evaporated to a solid which was recrystallised from ethyl acetate to give the desired product (0.45 g, 34%), m.p. 182—184°C. Found: C, 44.1%; H, 3.6; N, 28.0. $C_{13}H_{13}Cl_2N_7O$ requires C, 44.1; H, 3.7; N, 27.7%. NMR ($CDCl_3$) exhibited a methyl signal at 4.08 consistent with the 1-methyl isomer. m/e 354 ($M^+$).

### Example 2

2-(2,4-Dichlorophenyl)-1-(1,2,4-triazol-3-yl)-3-(1H, 1,2,4-triazol-1-yl)propan-2-ol, dioxalate

3-(2,4-Dichlorophenyl)-3-hydroxy-4-(1H-1,2,4-triazol-1-yl)butyrimidic acid, ethyl ester dihydrochloride (0.5 g) was converted to its free base by partitioning between methylene chloride (20 ml) and saturated sodium bicarbonate solution (5 ml). The organic layer was dried ($MgSO_4$) and evaporated to a gum (0.42 g) under reduced pressure at room temperature. Formyl hydrazine (0.1 g) and ethanol (2 ml) were added and the mixture was stirred at room temperature for 12 hours. The precipitate which formed was collected by filtration and added to toluene (25 ml) containing a trace of para-toluenesulphonic acid. The solution was heated under reflux for one hour and a toluene solution was then washed with saturated sodium bicarbonate solution (5 ml), dried over $MgSO_4$ and evaporated to a gum. The product was taken up in a mixture of diethyl ether and ethyl acetate and converted to its oxalate salt by the dropwise addition of a solution of oxalic acid in ethyl acetate. The resulting amorphous solid was collected and dried to yield the title compound (0.108 g, 27%). Hygroscopic. Found: C, 37.2; H, 3.2; N, 15.6. $C_{13}H_{12}Cl_2N_6O . 2C_2O_4H_2 . H_2O$ requires C, 38.0; H, 3.5; N, 15.6%.

*NMR* (δ, $CDCl_3$): 3.38 (d, J=1Hz, 1H), 3.95 (d, J=15Hz, 1H), 4.7 (d, J=15Hz, 1H), 5.0 (d, J=15Hz, 1H), 6.25 (bs, 1H), 7.05 (dd, J=9Hz, 2Hz, 1H), 7.27 (d, J=2Hz, 1H), 7.55 (d, J=9Hz, 1H), 7.77 (s, 1H), 7.9 (s, 1H), 8.15 (s, 1H).

### Example 3

2-(2,4-Dichlorophenyl)-1-(1-methyl-1,2,4-triazol-3--yl)-3-(1H-1,2,4-triazol-1-yl)propan-2-ol
2-(2,4-Dichlorophenyl)-1-(2-methyl-1,2,4-triazol-3-yl)-3-(1H-1-2,4-triazol-1-yl)propan-2-ol and
2-(2,4-Dichlorophenyl)-1-(4-methyl-1,2,4-triazol-3-yl)-3-(1H-1,2,4-triazol-1-yl)propan-2-ol

The product from Example 2, (0.18 g) as its free base, was stirred with methyl iodide (0.25 g) and potassium carbonate (0.2 g) in acetone (12 ml) at room temperature for 1 hour. The acetone was removed under reduced pressure and the residue taken up in chloroform, filtered and the solution applied to a silica column. Elution with methylene chloride initially containing 2%, rising to 6% isopropanol (by volume) and a trace of ammonia gave three components identified as the three possible N-methyl-triazolyl isomers.

Fraction 1: 45 mg (24%), m.p. 170—173°C; m/e 352 ($M^+$)

*NMR* (δ, $CDCl_3$): 3.18 (d, J=15Hz, 1H), 3.75 (s, 3H), 4.0 (d, J=15Hz, 1H), 4.85 (s, 2H), 6.54 (bs, 1H), 7.15 (dd, J=9Hz, 2Hz, 1H), 7.35 (d, J=2Hz, 1H), 7.65 (d, J=9Hz, 1H), 7.65 (s, 1H), 7.8 (s, 1H), 8.2 (s, 1H).

Fraction 2: 44 mg (19%) glass. m/e 270 (M+ — 82)

*NMR* (δ, $CDCl_3$): 3.14 (d, J=16Hz, 1H), 3.75 (s, 3H), 3.95 (d, J=16Hz, 1H), 4.85 (s, 2H), 6.0 (bs, 1H), 7.05 (dd, J=9Hz, 2Hz, 1H), 7.3 (d, J=2Hz, 1H), 7.55 (d, J=9Hz, 1H), 7.8 (bs, 2H), 8.15 (s, 1H).

Fraction 3: 8 mg (4%) m.p. 190—193°C m/e 270 ($M^+$ — 82)

*NMR* (δ, $CDCl_3$): 3.27 (d, J=16Hz, 1H), 3.55 (s, 3H), 3.85 (d, J=16Hz, 1H), 4.9 (s, 2H), 6.25 (bs, 1H), 7.17 (dd, J=9Hz, 2Hz, 1H), 7.37 (d, J=2Hz, 1H), 7.7 (d, J=9Hz, 1H), 7.8 (3, 1H), 7.9 (s, 1H), 8.15 (s, 1H).

Example 4

2-(2,4-Dichlorophenyl)-1-(imidazol-2-yl)-3-(1H-1,2,4-triazol-1-yl)propan-2-ol

A solution of 3-(2,4-dichlorophenyl)-3-hydroxy-4-(1H-1,2,4-triazol-1-yl)butyrimidic acid ethyl ester free base (546 mg), aminoacetaldehyde diethylacetal (234 mg) and acetic acid (210 mg) in tetrahydrofuran (4 ml) was heated under reflux for 2 hours. Hydrochloric acid (4 ml, 5N) was added and the mixture heated to 70°C for 30 minutes. The mixture was diluted with saturated sodium carbonate to pH 8 and extracted with ethyl acetate (3 × 20 ml). The organic extracts were dried over magnesium sulphate and evaporated to yield a gum which was chromatographed on silica eluting with a mixture of methylene chloride, isopropanol and concentrated ammonium hydroxide (85:15:1 by volume). Fractions containing the desired product were pooled and evaporated to yield a gum which solidified on trituration with diethyl ether (246 mg). Recrystallisation from isopropanol gave the title compound as colourless crystals m.p. 168—170°C. Found: C, 49.8; H, 3.9; N, 20.9. $C_{14}H_{13}Cl_2N_5O$ requires C, 49.7; H, 3.9; N, 20.7%.

Example 5

2-(2,4-Dichlorophenyl)-1-(1-methylimidazol-2-yl)-3-(1H-1,2,4-triazol-1-yl)propan-2-ol dimesylate

2-(2,4-Dichlorophenyl)-1-(imidazol-2-yl)-3-(1H-1,2,4-triazol-1-yl)propan-2-ol (80 mg), powdered anhydrous potassium carbonate (200 mg) and methyl iodide (200 mg) were stirred for 3 hours at room temperature in acetone (5 ml). The acetone was evaporated and the residue chromatographed on silica, eluting with a mixture of methylene chloride, isopropanol and concentrated ammonium hydroxide (95:5:1 by volume). Fractions containing the major product were combined and evaporated to a gum which was taken up in ethyl acetate (2 ml) and treated with a solution of methane sulphonic acid in diethyl ether. The precipitate of the dimesylate salt (70 mg) was collected and recrystallised from isopropanol to give the title compound as colourless crystals. m.p. 201—203°C. Found: C, 37.4; H, 4.3; N, 12.7. $C_{15}H_{15}Cl_2N_5O . 2CH_3SO_3H$ requires C, 37.5; H, 4.2; N, 12.9%.

Preparation 1

Preparation of 1-cyano-2-(2,4-dichlorophenyl)-3-(1H-1,2,4-triazol-1-yl)propan-2-ol

2-(2,4-Dichlorophenyl)-2-(1H-1,2,4-triazol-1-ylmethyl)oxirane (1.09 g) and sodium cyanide (0.6 g) in dimethylformamide (30 ml) were heated at 65—70°C for 1 hour. The reaction mixture was then cooled, poured into water (150 ml), and extracted with ethyl acetate (3 × 25 ml). The combined organic extracts were washed with saturated aqueous brine, dried ($Na_2SO_4$) and evaporated to dryness to give a pale yellow solid (0.76 g) which was triturated with ether. The residual solid was recrystallised from ether/methanol to give the title compound 285 mg (24%), m.p. 217—219°C. Found: C, 48.3; H, 3.4; N, 18.4. Calculated for $C_{12}H_{10}Cl_2N_4O$: C, 48.5; H, 3.4; N, 18.8%.

Preparation 2

Preparation of 3-(2,4-dichlorophenyl)-3-hydroxy-4-(1H-1,2,4-triazol-1-yl)butyrimidic acid, ethyl ester dihydrochloride

1-Cyano-2-(2,4-dichlorophenyl)-3-(1H-1,2,4-triazol-1-yl)propan-2-ol (1 g) was dissolved in dry ethyl alcohol (100 ml) and dry hydrogen chloride gas was bubbled in, at 0°C, for 10 minutes. The reaction mixture was then stirred at room temperature overnight, and then the solvent was decanted from the solid. The solid was then washed with dry ether and dried to yield the title compound, (1.15 g), m.p. 154—156°C. Found: C, 40.6; H, 4.4; N, 13.6. Calculated for $C_{14}H_{16}Cl_2N_4O_2 . 2HCl$: C, 40.4; H, 4.4; N, 13.5%.

Test Results

The compounds of the Examples were tested *in vivo* by oral administration to mice inoculated with a lethal infection of *Candida albicans* according to the procedures described herein. The dose levels providing 50% protection ($PD_{50}$) were as follows:

| Example | $PD_{50}$ (mg/kg/p.o.) |
|---|---|
| 1 | 0.1 |
| 2 | 2.2 |
| 3 (1) | 1.5 |
| (2) | 20 |
| (3) | 3.5 |
| 4 | 1.2 |
| 5 | 1.0 |

**Claims for the Contracting States: BE CH DE FR GB IT LI LU NL SE**

1. A compound of the formula:

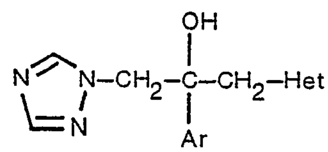

(I)

where

Ar is phenyl optionally substituted by 1 to 3 substituents each independently selected from F, Cl, Br, I, $CF_3$, $C_1$—$C_4$ alkyl and $C_1$—$C_4$ alkoxy; and

Het is a group of the formula:

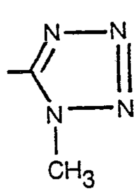
,
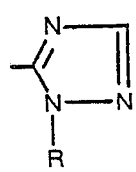
,
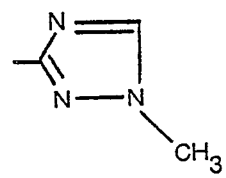

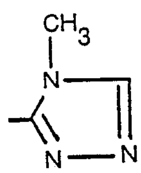
or
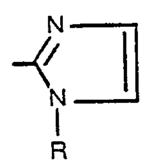

wherein R is H or $CH_3$;

and their pharmaceutically acceptable salts.

2. A compound according to claim 1 wherein Ar is 4-fluorophenyl, 4-chlorophenyl, 4-bromophenyl, 4-iodophenyl, 4-trifluoromethylphenyl, 2-chlorophenyl, 2,4-dichlorophenyl, 2,4-difluorophenyl, 2,5-difluorophenyl, 2-chloro-4-fluorophenyl, 2,4,6-trifluorophenyl or 4-bromo-2,5-difluorophenyl.

3. A compound according to claim 2 wherein Ar is 2,4-dichlorophenyl.

4. A compound according to any one of claims 1 to 3 wherein Het is 1-methyltetrazol-5-yl.

5. 2-(2,4-dichlorophenyl)-1-(1-methyltetrazol-5-yl)-3-(1H-1,2,4-triazol-1-yl)propan-2-ol;

6. A pharmaceutical composition comprising a compound as claimed in any one of claims 1 to 5, or a pharmaceutically acceptable salt thereof, together with a pharmaceutically acceptable diluent or carrier.

7. A process for preparing a compound of the formula (I) as claimed in claim (I) characterised by

(a) methylating a compound of the formula:

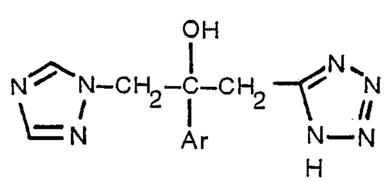

(IV)

wherein Ar is as defined in claim 1,
and separating the desired compound of formula (I) wherein Het is 1-methyl-tetrazol-5-yl or
(b) reacting a compound of the formula:

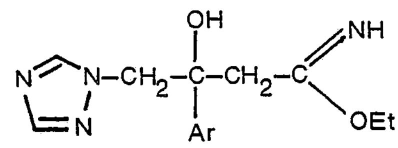

(V)

wherein Ar is as defined in claim 1, with formyl hydrazine to obtain the compound of formula (I) wherein Het is 1,2,4-triazol-3-yl and optionally methylating and separating the isomers to obtain the compounds of formula (I) wherein Het is 1-methyl-1,2,4-triazol-3-yl, 2-methyl-1,2,4-triazol-3-yl or 4-methyl-1,2,4-triazol-3-yl or

(c) reacting a compound of the formula (V) with aminoacetaldehyde followed by treatment with acid to obtain the compound of formula (I) wherein Het is imidazol-2-yl and optionally methylating to obtain the compound of formula (I) wherein Het is 1-methyl-imidazol-2-yl

and in each case optionally forming a pharmaceutically acceptable salt of the product.

8. A compound of the formula (I) as claimed in any one of claims 1 to 5 or a pharmaceutically acceptable salt thereof for use in treating fungal infections in humans and other animals.

**Claims for the Contracting State: AT**

1. A compound of the formula:

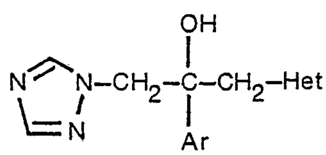

(I)

where
Ar is phenyl optionally substituted by 1 to 3 substituents each independently selected from F, Cl, Br, I, $CF_3$, $C_1$—$C_4$ alkyl and $C_1$—$C_4$ alkoxy; and
Het is a group of the formula:

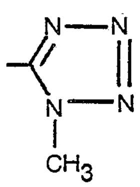
,
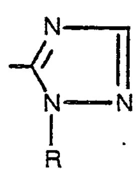
,
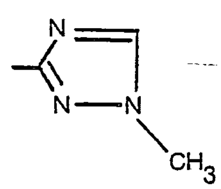

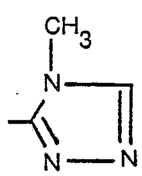
or
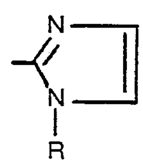

or a pharmaceutically acceptable salt thereof, characterised by methylating, as appropriate, a compound of the formula (I) in which Het is selected from:—

(a) tetrazol-5-yl,
(b) 1,2,4-triazol-3-yl, and
(c) imidazol-2-yl,

followed by, in each case, optionally forming a pharmaceutically acceptable salt of the product, and said reaction (b) being followed by separating the resulting isomers, if desired, to isolate products in which Het is 1-methyl-1,2,4-triazol-3-yl, 2-methyl-1,2,4-triazol-3-yl, or 4-methyl-1,2,4-triazol-3-yl.

2. A process according to claim 1, characterised in that Het is tetrazol-5-yl, and wherein said methylation is carried out in the presence of potassium carbonate.

3. A process for preparing a compound of the formula (I) as defined in claim 1 wherein Het is 1,2,4-triazol-3-yl, 1-methyl-1,2,4-triazol-3-yl, 2-methyl-1,2,4-triazol-3-yl or 4-methyl-1,2,4-triazol-3-yl, or a pharmaceutically acceptable salt thereof, characterised by reacting a compound of the formula:—

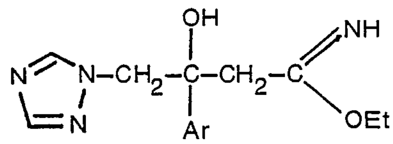

(V)

wherein Ar is as defined in claim 1, with formyl hydrazine followed by treatment with acid to obtain the compound of formula (I) wherein Het is 1,2,4-triazol-3-yl and optionally methylating and separating the isomers to obtain the compounds of formula (I) wherein Het is 1-methyl-1,2,4-triazol-3-yl, 2-methyl-1,2,4-triazol-3-yl or 4-methyl-1,2,4-triazol-3-yl, and also optionally forming a pharmaceutically acceptable salt of the product.

4. A process for preparing a compound of the formula (I) as defined in claim 1 in which Het is imidazol-2-yl or 1-methyl-imidazol-2-yl, or a pharmaceutically acceptable salt thereof, characterised by reacting a compound of the formula (V) as defined in claim 3 with aminoacetaldehyde, preferably as its diethyl acetal, followed by treatment with acid to obtain the compound of formula (I) wherein Het is imidazol-2-yl and optionally methylating to obtain the compound of formula (I) wherein Het is 1-methyl-imidazol-2-yl; and also optionally forming a pharmaceutically acceptable salt of the product.

5. A process according to any one of the preceding claims characterised in that Ar is 4-fluorophenyl, 4-chlorophenyl, 4-bromophenyl, 4-iodophenyl, 4-trifluoromethylphenyl, 2-chlorophenyl, 2,4-dichlorophenyl, 2,4-difluorophenyl, 2,5-difluorophenyl, 2-chloro-4-fluorophenyl, 2,4,6-trifluorophenyl or 4-bromo-2,5-difluorophenyl.

6. A process according to claim 5 characterised in that Ar is 2,4-dichlorophenyl.

**Patentansprüche für die Vertragsstaaten: BE CH DE FR GB IT LI LU NL SE**

1. Eine Verbindung der Formel:

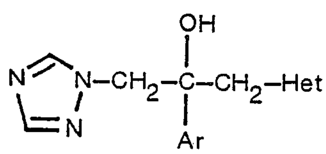

(I)

worin

Ar Phenyl gegebenenfalls substituiert durch 1 bis 3 Substituenten, jeweils unabhängig voneinander ausgewählt aus F, Cl, Br, J, $CF_3$, $C_1$—$C_4$-Alkyl und $C_1$—$C_4$-Alkoxy, bedeutet und

Het eine Gruppe der Formel

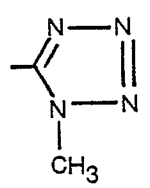
,
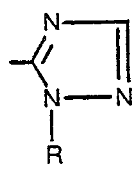
,
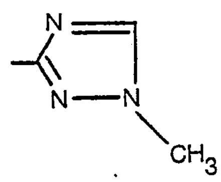

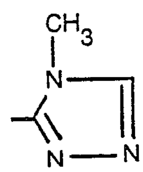
oder
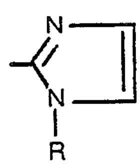

ist, wobei R H oder $CH_3$ darstellt, und deren pharmazeutisch annehmbare Salze.

2. Verbindung nach Anspruch 1, worin Ar 5-Fluorphenyl, 4-Chlorphenyl, 4-Bromphenyl, 4-Jodphenyl, 4-Trifluormethylphenyl, 2-Chlorphenyl, 2,4-Dichlorphenyl, 2,4-Difluorphenyl, 2,5-Difluorphenyl, 2-Chlor-4-fluorphenyl, 2,4,6-Trifluorphenyl oder 4-Brom-2,5-difluorphenyl ist.

3. Verbindung nach Anspruch 2, worin Ar 2,4-Dichlorphenyl ist.

4. Verbindung nach einem der Ansprüche 1 bis 3, worin Het 1-Methyltetrazol-5-yl ist.

5. 2-(2,4-Dichlorphenyl)-1-(1-methyltetrazol-5-yl)-3-(1H-1,2,4-triazol-1-yl)-propan-2-ol.

6. Pharmazeutische Zusammensetzung, die eine Verbindung, wie in einem der Ansprüche 1 bis 5 beansprucht, oder ein pharmazeutisch annehmbares Salz hievon zusammen mit einem pharmazeutisch annehmbaren Verdünnungsmittel oder Träger umfaßt.

7. Verfahren zum Herstellen einer Verbindung der Formel (I), wie in Anspruch 1 beansprucht, gekennzeichnet durch

(a) Methylieren einer Verbindung der Formel:

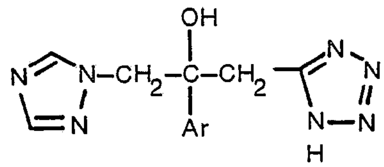

(IV)

worin Ar wie in Anspruch 1 definiert ist, und Abtrennen der gewünschten Verbindung der Formel (I), worin Het 1-Methyl-tetrazol-5-yl bedeutet, oder

(b) Umsetzen einer Verbindung der Formel

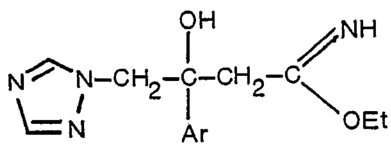

(V)

worin Ar wie in Anspruch 1 definiert ist, mit Formylhydrazin under Erzielung einer Verbindung der Formel (I), worin Het 1,2,4-Triazol-3-yl ist, und gegebenenfalls Methylieren und Trennen der Isomeren, wobei Verbindung der Formel (I) erhalten werden, worin Het 1-Methyl-1,2,4-triazol-3-yl, 2-Methyl-1,2,4-triazol-3-yl oder 4-Methyl-1,2,4-triazol-3-yl bedeutet, oder

(c) Umsetzen einer Verbindung der Formel (V) mit Aminoacetaldehyd, gefolgt von Behandlung mit einer Säure, wobei die Verbindung der Formel (I) erhalten wird, worin Het Imidazol-2-yl ist, und gegebenenfalls Methylieren zum Erzielen der Verbindung der Formel (I), worin Het 1-Methylimidazol-2-yl bedeutet, und im jedem Fall gegebenenfalls Bilden eines pharmazeutisch annehmbaren Salzes des Produktes.

8. Verbindung der Formel (I), wie in einem der Ansprüche 1 bis 5 beansprucht, oder ein pharmazeutisch annehmbares Salz hievon zur Verwendung bei der Behandlung von Pilzinfektionen bei Menschen und Tieren.

**Patentansprüche für den Vertragsstaat: AT**

1. Verfahren zum Herstellen einer Verbindung der Formel:

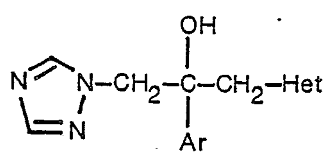

(I)

worin

Ar Phenyl gegebenenfalls substituiert durch 1 bis 3 Substituenten, jeweils unabhängig ausgewählt aus F, Cl, Br, J, $CF_3$, $C_1$—$C_4$-Alkyl und $C_1$—$C_4$-Alkoxy, bedeutet und

Het eine Gruppe der Formel

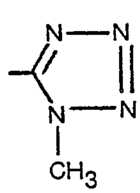 , 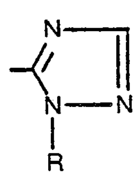 , 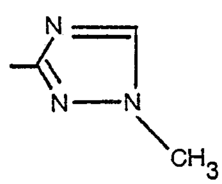

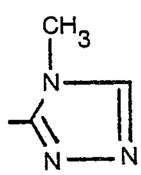 oder 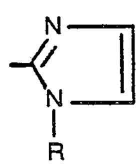

ist, oder eines pharmazeutisch annehmbaren Salzes hievon, gekennzeichnet durch, wie geeignet, Methylieren einer Verbindung der Formel (I), worin Het ausgewählt ist aus

(a) Tetrazol-5-yl,
(b) 1,2,4-Triazol-3-yl und
(c) Imidazol-2-yl,

in jedem Fall gegebenenfalls gefolgt vom Bilden eines pharmazeutisch annehmbaren Salzes des Produktes, und

wobei der genannten Reaktion (b), wenn gewünscht, die Trennung der erhaltenen Isomeren zum Isolieren von Produkten, worin Het 1-Methyl-1,2,4-triazol-3-yl, 2-Methyl-1,2,4-triazol-3-yl oder 4-Methyl-1,2,4-triazol-3-yl bedeutet, folgt.

2. Verfahren nach Anspruch 1, dadurch gekennzeichnet, daß Het Tetrazol-5-yl ist und worin die genannte Methylierung in Anwesenheit von Kaliumcarbonat durchgeführt wird.

3. Verfahren zum Herstellen einer Verbindung der Formel (I), wie in Anspruch 1 definiert, worin Het 1,2,4-Triazol-3-yl, 1-Methyl-1,2,4-triazol-3-yl, 2-Methyl-1,2,4-triazol-3-yl oder 4-Methyl-1,2,4-triazol-3-yl bedeutet, oder eines pharmazeutisch annehmbaren Salzes hievon, gekennzeichnet durch das Umsetzen einer Verbindung der Formel:

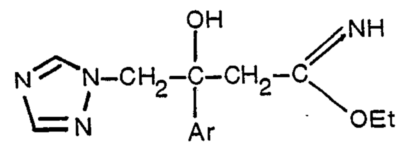

(V)

worin Ar wie in Anspruch 1 definiert ist, mit Formylhydrazin, gefolgt von Behandlung mit einer Säure zum Erzielen der Verbindung der Formel (I), worin Het 1,2,4-Triazol-3-yl bedeutet, und gegebenenfalls Methylierung und Trennung der Isomeren zum Erzielen der Verbindungen der Formel (I), worin Het 1-Methyl-1,2,4-triazol-3-yl, 2-Methyl-1,2,4-triazol-3-yl oder 4-Methyl-1,2,4-triazol-3-yl bedeutet, und auch gegebenenfalls Bildung eines pharmazeutisch annehmbaren Salzes des Produktes.

4. Verfahren zum Herstellen einer Verbindung der Formel (I), wie in Anspruch 1 definiert, worin Het Imidazol-2-yl oder 1-Methylimidazol-2-yl bedeutet, oder eines pharmazeutisch annehmbaren Salzes hievon, gekennzeichnet durch Umsetzen einer Verbindung der Formel (V), wie in Anspruch 3 definiert, mit Aminoacetaldehyd, vorzugsweise als sein Diäthylacetal, gefolgt von Behandlung mit einer Säure zum Erzielen der Verbindung der Formel (I), worin Het Imidazol-2-yl bedeutet, und gegebenenfalls Methylieren zum Erzielen der Verbindung der Formel (I), worin Het 1-Methylimidazol-2-yl bedeutet; und auch gegebenenfalls Bilden eines pharmazeutisch annehmbaren Salzes des Produktes.

5. Verfahren nach einem der vorhergehenden Ansprüche, dadurch gekennzeichnet, daß Ar 4-Fluorphenyl, 4-Chlorphenyl, 4-Bromphenyl, 4-Jodphenyl, 4-Trifluormethylphenyl, 2-Chlorphenyl, 2,4-Dichlorphenyl, 2,4-Difluorphenyl, 2,5-Difluorphenyl, 2-Chlor-4-fluorphenyl, 2,4,6-Trifluorphenyl oder 4-Brom-2,5-difluorphenyl bedeutet.

6. Verfahren nach Anspruch 5, dadurch gekennzeichnet, daß Ar 2,4-Dichlorphenyl bedeutet.

**Revendications pour les Etats contractants: BE CH DE FR GB IT LI LU NL SE**

1. Composé de formule:

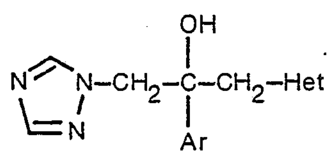

(I)

dans laquelle

Ar est un groupe phényle éventuellement substitué par 1 à 3 substituants choisis chacun, indépendamment, entre F, Cl, Br, I, $CF_3$, alkyle en $C_1$ à $C_4$ et alkoxy en $C_1$ à $C_4$; et

Het est un groupe de formule:

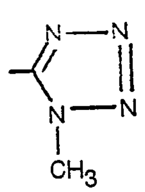
,
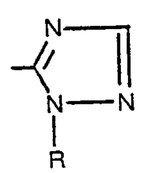
,
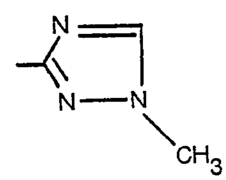

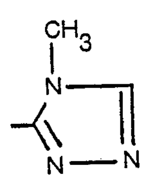
ou
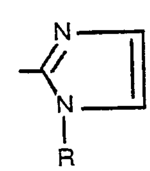

dans laquelle Ar représente H ou $CH_3$;
et ses sels pharmaceutiquement acceptables.

2. Composé suivant la revendication 1, dans lequel Ar est un groupe 4-fluorophényle, 4-chlorophényle, 4-bromophényle, 4-iodophényle, 4-trifluorométhylphényle, 2-chlorophényle, 2,4-dichlorophényle, 2,4-difluorophényle, 2,5-difluorophényle, 2-chloro-4-fluorophényle, 2,4,6-trifluorophényle ou 4-bromo-2,5-difluorophényle.

3. Composé suivant la revendication 2, dans lequel Ar est le groupe 2,4-dichlorophényle.

4. Composé suivant l'une quelconque des revendications 1 à 3, dans lequel Het est le groupe 1-méthyltétrazole-5-yle.

5. Le 2-(2,4-dichlorophényl)-1-(1-méthyltétrazole-5-yl)-3-(1H-1,2,4-triazole-1-yl)propane-2-ol.

6. Composition pharmaceutique comprenant un composé suivant l'une quelconque des revendications 1 à 5 ou un sel pharmaceutiquement acceptable de ce composé, en association avec un diluant ou support acceptable du point de vue pharmaceutique.

7. Procédé de préparation d'un composé de formule (I) suivant la revendication 1, caractérisé par
(a) la méthylation d'un composé de formule:

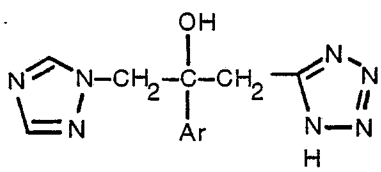

(IV)

dans laquelle Ar est tel que défini dans la revendication 1,
et la séparation du composé désiré de formule (I) dans laquelle Het est le groupe 1-méthyltétrazole-5-yle, ou

(b) la réaction d'un composé de formule:

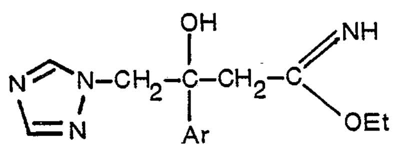

(V)

dans laquelle Ar est tel que défini dans la revendication 1, avec la formylhydrazine pour obtenir le composé de formule (I) dans laquelle Het est le groupe 1,2,4-triazole-3-yle et, à titre facultatif, la méthylation et la séparation des isomères pour obtenir les composés de formule (I) dans laquelle Het est un groupe 1-méthyl-1,2,4-triazole-3-yle, 2-méthyl-1,2,4-triazole-3-yle ou 4-méthyl-1,2,4-triazole-3-yle ou

(c) la réaction d'un composé de formule (V) avec l'amino-acétaldéhyde, suivie d'un traitement avec un acide pour obtenir le composé de formule (I) dans laquelle Het est le groupe imidazole-2-yle et, à titre facultatif, la méthylation en vue d'obtenir le composé de formule (I) dans laquelle Het est le groupe 1-méthyl-imidazole-2-yle et dans chaque cas, à titre facultatif, la formation d'un sel pharmaceutiquement acceptable du produit.

8. Composé de formule (I) suivant l'une quelconque des revendications 1 à 5 ou un sel pharmaceutiquement acceptable de ce composé, destiné à être utilisé au traitement d'infections fongiques chez des êtres humains et d'autres animaux.

**Revendications pour l'Etat contractant: AT**

1. Procédé de préparation d'un composé de formule:

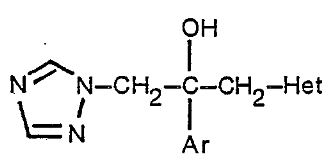

(I)

dans laquelle

Ar est un groupe phényle éventuellement substitué par 1 à 3 substituants choisis chacun, indépendamment, entre F, Cl, Br, I, $CF_3$, alkyle en $C_1$ à $C_4$ et alkoxy en $C_1$ à $C_4$; et

Het est un groupe de formule:

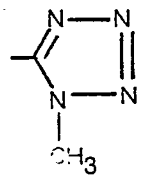 , 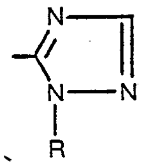 , 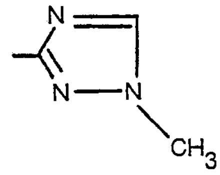 , 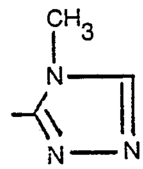 ou 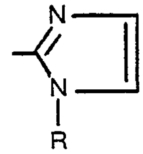

ou d'un sel pharmaceutiquement acceptable de ce composé, caractérisé par la méthylation, selon le cas approprié, d'un composé de formule (I) dans laquelle Het est choisi entre:

(a) le groupe tétrazole-5-yle,

(b) le groupe 1,2,4-triazole-3-yle, et

(c) le groupe imidazole-2-yle,

suivie, dans chaque cas, de la formation facultative d'un sel pharmaceutiquement acceptable du produit, ladite réaction (b) étant suivie de la séparation éventuelle des isomères résultants pour isoler des produits dans lesquels Het est un groupe 1-méthyl-1,2,4-triazole-3-yle, 2-méthyl-1,2,4-triazole-3-yle ou 4-méthyl-1,2,4-triazole-3-yle.

2. Procédé suivant la revendication 1, caractérisé en ce que Het est le groupe tétrazole-5-yle et ladite méthylation est conduite en présence de carbonate de potassium.

3. Procédé de préparation d'un composé de formule (I) suivant la revendication 1, dans lequel Het est le groupe 1,2,4-triazole-3-yle, 1-méthyl-1,2,4-triazole-3-yle, 2-méthyl-1,2,4-triazole-3-yle ou 4-méthyl-1,2,4-triazole-3-yle, ou d'un sel pharmaceutiquement acceptable de ce composé, caractérisé par la réaction d'un composé de formule:

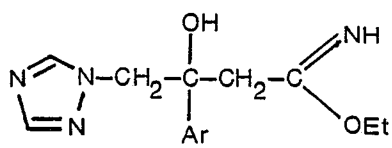 (V)

dans laquelle Ar est tel que défini dans la revendication 1, avec la formylhydrazine, suivie d'un traitement avec un acide pour obtenir le composé de formule (I) dans laquelle Het est le groupe 1,2,4-triazole-3-yle et, à titre facultatif, de la méthylation et de la séparation des isomères pour obtenir les composés de formule (I) dans laquelle Het est le groupe 1-méthyl-1,2,4-triazole-3-yle, 2-méthyl-1,2,4-triazole-3-yle ou 4-méthyl-1,2,4-triazole-3-yle, avec formation éventuelle d'un sel pharmaceutiquement acceptable du produit.

4. Procédé de préparation d'un composé de formule (I) tel que défini dans la revendication 1, dans lequel Het est le groupe imidazole-2-yle ou 1-méthylimidazole-2-yle, ou d'un sel pharmaceutiquement acceptable de ce composé, caractérisé par la réaction d'un composé de formule (V) tel que défini dans la revendication 3 avec l'amino-acétaldéhyde, de préférence sous la forme de son diéthylacétal, suivie d'un traitement avec un acide pour obtenir le composé de formule (I) dans laquelle Het est le groupe imidazole-2-yle et, le cas échéant, d'une méthylation pour obtenir le composé de formule (I) dans laquelle Het est le groupe 1-méthylimidazole-2-yle;

avec éventuellement formation d'un sel pharmaceutiquement acceptable du produit.

5. Procédé suivant l'une quelconque des revendications prédédentes, caractérisé en ce que Ar est le groupe 4-fluorophényle, 4-chlorophényle, 4-bromophényle, 4-iodophényle, 4-trifluorométhylphényle, 2-chlorophényle, 2,4-dichlorophényle, 2,4-difluorophényle, 2,5-difluorophényle, 2-chloro-4-fluorophényle, 2,4,6-trifluorophényle ou 4-bromo-2,5-difluorophényle.

6. Procédé suivant la revendication 5, caractérisé en ce que Ar est le groupe 2,4-dichlorophényle.